Europäisches Patentamt

European Patent Office

Office européen des brevets

(19)

(11) Numéro de publication : **0 300 012 B1**

(12) # FASCICULE DE BREVET EUROPEEN

(45) Date de publication du fascicule du brevet :
**28.08.91 Bulletin 91/35**

(51) Int. Cl.⁵ : **A61M 1/12, F04B 43/06**

(21) Numéro de dépôt : **88901281.1**

(22) Date de dépôt : **27.01.88**

(86) Numéro de dépôt international :
**PCT/FR88/00043**

(87) Numéro de publication internationale :
**WO 88/05313 28.07.88 Gazette 88/17**

(54) **PROTHESE CARDIAQUE TOTALE PERFECTIONNEE.**

(30) Priorité : **27.01.87 FR 8700908**

(43) Date de publication de la demande :
**25.01.89 Bulletin 89/04**

(45) Mention de la délivrance du brevet :
**28.08.91 Bulletin 91/35**

(84) Etats contractants désignés :
**AT BE CH DE FR GB IT LI LU NL SE**

(56) Documents cités :
**EP-A-00 141 30**
**EP-A-00 747 33**
**FR-A- 2 572 491**
**GB-A- 1 079 211**

(73) Titulaire : **Lapeyre, Didier, Dr.**
**Chaignes**
**F-27120 Pacy sur Eure (FR)**

(72) Inventeur : **VERAGEN, René**
**13, rue Camille-Chevillard**
**F-78400 Chatou (FR)**

(74) Mandataire : **Ores, Irène et al**
**CABINET ORES 6, Avenue de Messine**
**F-75008 Paris (FR)**

## Description

L'invention a pour objet une prothèse cardiaque totale perfectionnée selon le préambule de la revendication 1.

Elle vise, plus particulièrement, une prothèse cardiaque totale de faible encombrement, à source d'activation unique pour les ventricules droit et gauche, présentant une géométrie fonctionnelle qui se rapproche autant que faire se peut de la géométrie fonctionnelle du coeur naturel et qui reproduise les lois de fonctionnement hémodynamique du coeur naturel, en particulier, la loi de STARLING et la loi de SARNOFF.

Une prothèse de ce type qui s'est révélée d'un fonctionnement satisfaisant, aussi bien lors d'essais in vitro que lors d'essais in vivo sur l'animal est divulguée dans FR-A-2446631. Elle comporte un boîtier implantable dans la cavité péricardique et dont la géométrie reproduit de très près la géométrie d'un coeur naturel avec à l'intérieur de ce boîtier un dispositif moteur essentiellement constitué par deux membranes dont l'une travaille à l'élongation dans un espace définissant le ventricule droit et dont l'autre travaille à la déformation dans un espace définissant le ventricule gauche, le dispositif moteur agissant, respectivement, sur des sacs à sang enfermés dans les espaces ventriculaires droit et gauche. Les sacs à sang sont propres à être reliés aux vaisseaux du réseau circulatoire d'un patient par des valves montées dans des orifices valvulaires ménagés dans le boîtier de la prothèse, celle-ci étant actionnée par des moyens d'activation du dispositif moteur comme une source d'énergie pneumatique extra-corporelle ou un moteur électrique à pile implantable, ou un moteur électrique commandé par une source d'énergie nucléaire, etc..., avec des moyens à boucles d'asservissement de régulation du débit sanguin.

Comme décrit dans le Brevet précité, la membrane du dispositif moteur du ventricule droit de la prothèse, à laquelle est associé un support, est une membrane en un matériau élastomère soumise à des allongements alternatifs au cours d'un très grand nombre de cycles de déformation en élongation, de sorte que peuvent apparaître, après plusieurs dizaines de millions d'alternances, des altérations comme des micro-fissures qui, à la longue, peuvent entraîner sa destruction.

Aussi le problème se pose-t-il de fournir une prothèse cardiaque totale du type de celle décrite ci-dessus, dont la membrane du ventricule droit ait une durée de vie aussi longue que possible, en tout cas supérieure à deux cent cinquante millions de cycles et qui, cependant, permette de satisfaire aux autres conditions imposées au dispositif moteur de la prothèse, en particulier assurer à chaque pulsation l'éjection d'un volume compris entre 60 et 90 ml de sang. Etant donné, d'une part, que pour une membrane d'une certaine surface ce volume utile est directement lié à l'allongement de la membrane et que, d'autre part, l'allongement maximum conditionne le nombre de cycles auxquels peut être soumise ladite membrane sans apparition de micro-fissures, le problème posé est alors celui de fournir dans le dispositif moteur de la prothèse un agencement de membrane procurant la variation de volume souhaité pour un allongement aussi faible que possible.

Ce problème est résolu, selon l'invention, par le fait que la membrane élastique du ventricule droit et la pièce de support qui lui est associée sont montées dans le boîtier de la prothèse à l'aide de moyens agencés de manière telle que le mouvement de ladite membrane à l'élongation (systole) est décomposé en deux phases, dont l'une est un déplacement sans allongement et dont l'autre, seulement, est accompagnée d'un allongement tandis que le mouvement inverse (diastole) comprend d'abord une phase de rétraction suivie d'un déplacement sans modification de forme de ladite membrane et de la pièce support qui lui est associée.

Avec une telle disposition, l'allongement de la membrane du ventricule droit du dispositif moteur est limité à une valeur d'élongation inférieure à 8 à 10% de sorte que les efforts de fatigue sont considérablement réduits avec, pour conséquence, un maintien de l'intégrité de la membrane pour au moins deux cent cinquante millions de cycles de fonctionnement.

Dans une forme de réalisation, les moyens procurant la décomposition en deux phases du mouvement de la membrane du ventricule droit comprennent au moins un soufflet propre à être relié à la source d'énergie pneumatique et sur lequel sont fixées la membrane et la pièce de support qui lui est associée.

Dans un mode d'exécution, le soufflet est du type à déplacement parallèle.

Dans une variante, le soufflet est du type à déplacement pendulaire.

Dans l'un et l'autre mode d'exécution, l'invention prévoit que le soufflet soit lui-même réalisé en un matériau élastique et que son extrémité, distante de celle de fixation de la membrane et de la pièce support qui lui est associée, soit fixée par sertissage à une cloison étanche séparant les espaces ventriculaires droit et gauche de la prothèse, cloison sur laquelle est également fixée la membrane travaillant à la déformation du ventricule gauche.

Selon une autre caractéristique de l'invention, la phase de déplacement sans allongement de la membrane travaillant à l'élongation du ventricule droit est limitée, à l'élongation de ladite membrane (systole), par butée sur un organe d'arrêt de l'extrémité mobile du soufflet sur laquelle est attachée ladite membrane et la pièce de support qui lui est associée.

Une telle organisation du ventricule droit permet en outre de réduire la puissance motrice de la source d'énergie pneumatique, par rapport à celle de la prothèse connue, en raison de la moindre quantité d'énergie nécessaire pour l'actionnement de la membrane dudit ventricule dont on prévoit complémentairement d'aider le mouvement inverse de retour à la forme initiale qui lui est donnée par moulage —, qui est celle qui correspond au début de systole —, par une légère dépression pneumatique.

Au bon fonctionnement de la prothèse contribue la présence d'un lubrifiant sec ou liquide entre les sacs à sang, le boîtier de la prothèse, les membranes du dispositif moteur et les moyens associés à la membrane du ventricule droit pour la décomposition du mouvement de celle-ci en deux phases distinctes.

La forme générale du boîtier et celle des espaces ventriculaires droit et gauche sont proches de celles de la prothèse selon le Brevet français précité, de sorte que lesdits espaces ventriculaires à dimensions croissantes, de la pointe vers la base de la prothèse, favorisent une bonne orientation des vecteurs vitesse du sang mis en mouvement vers les orifices valvulaires de sortie, sans zone de stase.

Le mode d'exécution a soufflet pendulaire accroît encore l'effet favorable de la géométrie d'ensemble de la prothèse par le fait qu'elle provoque d'abord le vidage de la pointe du sac à sang du ventricule droit et cela à une plus grande vitesse que le vidage du reste du sac, — en raison du plus grand éloignement de cette pointe de la charnière d'articulation du mouvement pendulaire —, sans modification toutefois de la vitesse du sang à l'orifice valvulaire, d'une part, et, d'autre part, la poursuite dans les conditions habituelles du vidage du sac lorsque, à la fin de la première phase de mouvement de la membrane du ventricule droit, celle-ci commence à travailler à l'élongation, uniquement.

L'invention sera bien comprise par la description qui suit, faite à titre d'exemple et en référence au dessin annexé dans lequel :

— la figure 1 est une vue schématique, en coupe longitudinale, d'une prothèse cardiaque totale conforme à l'invention ;

— la figure 2 est une vue analogue à celle de la figure 1 mais pour une autre condition du dispositif moteur ;

— la figure 3 est une vue schématique en coupe transversale de la prothèse dans la condition montrée sur la figure 1 ;

— la figure 4 est une vue en coupe transversale de la prothèse dans la condition montrée sur la figure 2 ;

— la figure 5 est une vue analogue à celle de la figure 1 mais pour une autre forme de réalisation;

— la figure 6 est une vue de cette forme de réalisation mais pour une autre condition du dispositif moteur ;

— la figure 6a est une vue de détail des réalisations montrées sur les figures 1 à 4 et 5 et 6 ;

— la figure 7 est une vue schématique d'un dispositif moteur de prothèse perfectionnée selon l'invention ;

— la figure 8 est une vue de détail ;

— la figure 9 est une vue schématique en perspective du boîtier de la prothèse conforme à l'invention comportant quatre orifices valvulaires.

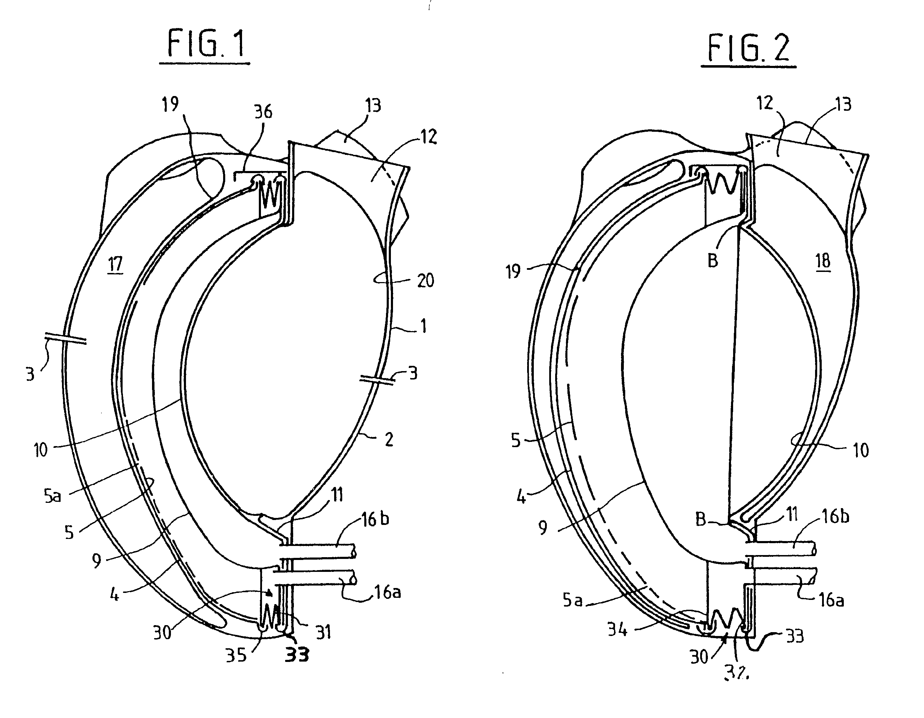

On se réfère d'abord aux figures 1 et 2 relatives à une première forme de réalisation d'une prothèse cardiaque totale selon l'invention. Celle-ci comprend un boîtier réalisé en un matériau non toxique à l'égard des tissus environnants, comme un polyuréthanne, avec une partie supérieure et une partie inférieure réunies l'une à l'autre d'une façon étanche par des moyens appropriés, par exemple des clips 3 placés dans un plan perpendiculaire aux directions de travail du dispositif moteur. Celui-ci comprend une première membrane 4, en matériau du type élastomère, qui travaille à l'élongation et dont la surface est de l'ordre d'au moins 150 cm² pour une prothèse d'un encombrement total d'environ 125 mm mais dont la surface peut être de l'ordre d'au moins 220 cm² pour une prothèse de plus grandes dimensions, par exemple d'un encombrement total de 150 mm.

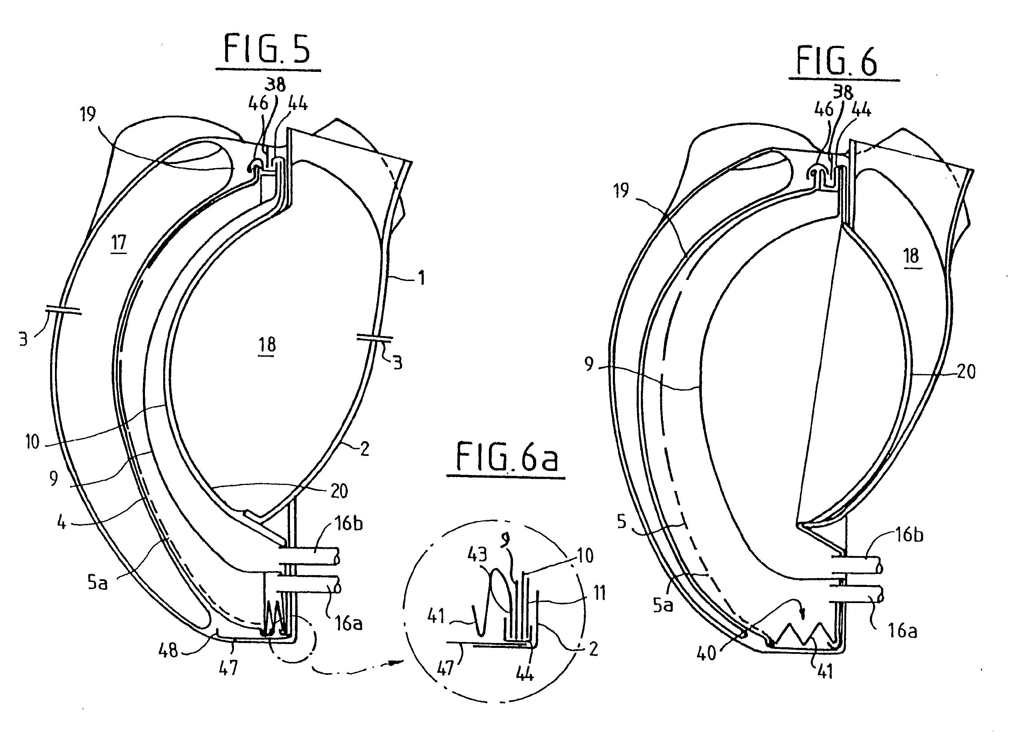

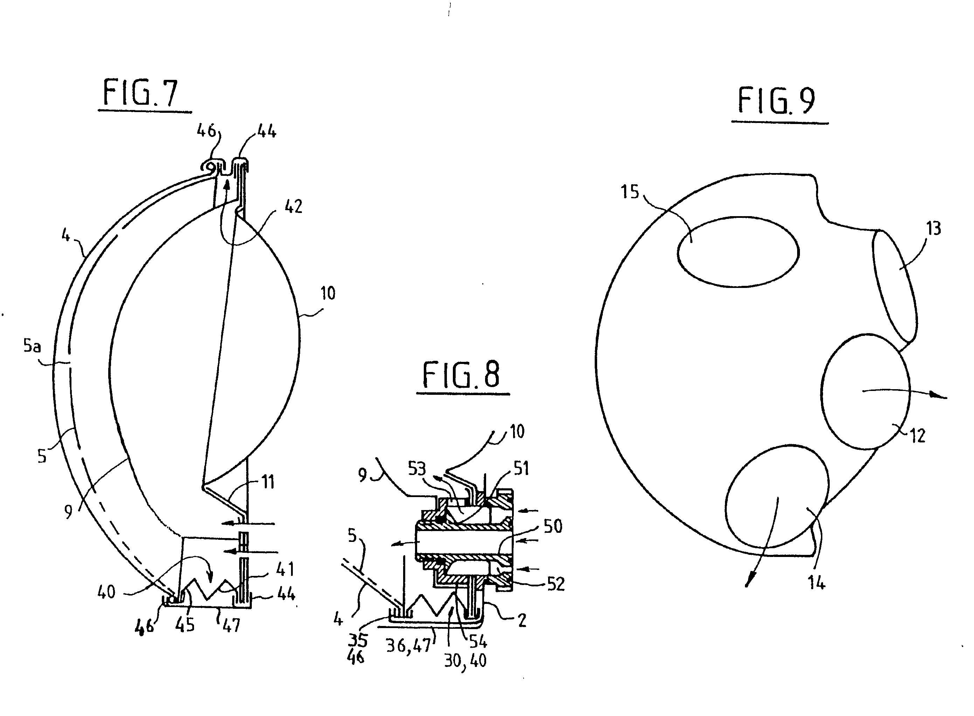

La membrane 4 délimite avec la face interne de la partie droite du boîtier qui lui fait face le ventricule droit de la prothèse, d'un volume d'environ 250 cm³ et dans lequel est logé un sac à sang 19 relié aux orifices valvulaires pulmonaire et tricuspide, 14 et 15, respectivement, figure 9, munis de valves, non représentées. Dans sa condition de repos, c'est-à-dire en absence totale d'élongation, la membrane 4 repose sur une pièce de support 5 constituée par un voile mince en matériau rigide, par exemple en métal ou en une matière plastique armée ou non armée, avantageusement percé de trous 5a et dont la forme est exactement celle de la membrane 4 qui l'épouse totalement dans sa condition non tendue. Comme bien montré sur les figures 1 et 5 la membrane 4 présente, au repos, une forme d'allure ovoïde, à rayons de courbure relativement grands en partie haute, sur le dessin, c'est-à-dire vers la base de la prothèse où sont prévus les orifices d'entrée 15 et de sortie 14 du sang et à rayons de courbure plus faibles vers la pointe de la prothèse, en partie basse sur le dessin, cette forme ovoïde de la membrane combinée à la forme de la face interne de la partie droite de la paroi du boîtier conférant à la cavité ventriculaire droite 17 une forme qui s'élargit régulièrement dans les trois directions de l'espace, de la pointe vers la base de la prothèse.

Les orifices 5a dans le voile de soutien 5 dont la densité est plus forte dans la partie de pointe que dans le reste dudit voile, comme montré schématiquement sur la figure 7, par exemple, sont prévus pour permettre l'action sur la membrane 4 d'un fluide sous pression distribué par une source d'énergie pneumatique reliée à la prothèse, en soi connue, dont la tubulure d'entrée et de sortie est montrée en 16a pour le ventricule droit et en 16b pour le ventricule gauche. Ce dernier, référencé 18, occupe un volume sensiblement égal à celui du ventricule droit et est limité par la face interne de la paroi gauche du boîtier 1, 2 et une cloison 9 qui s'étend sur toute la hauteur de la prothèse, de la pointe à la base de cette dernière.

La structure du ventricule gauche, qui est très proche de celle décrite dans le Brevet français précité, FR-A-2446631, comporte en tant qu'organe moteur une membrane mince 10 qui travaille à la déformation (et non à l'élongation comme la membrane 4) de part et d'autre d'un plan B-B incliné sur le plan axial longitudinal de la prothèse. La membrane 10 est fixée latéralement sur un support 11 en matériau rigide, par exemple en acier inoxydable qui limite sa déformation pour empêcher l'écrasement complet, en phase de systole, d'un sac à sang 20 logé dans l'espace ventriculaire 18 et qui présente, vers la base de la prothèse, les entrées et sorties de sang correspondant aux orifices valvulaires 12 et 13 du boîtier, c'est-à-dire les orifices aortique et mitral, respectivement. L'espace ventriculaire gauche 18 est lui aussi de forme progressivement croissante dans les trois directions de l'espace, de la pointe vers la base de la prothèse, une telle disposition ayant pour effet de favoriser des écoulements laminaires sans turbulence et d'éliminer les zones de vitesse nulle dans ledit espace tout en orientant de façon favorable les vecteurs vitesse du sang mis en mouvement vers l'aorte.

Selon l'invention, la membrane 4 du ventricule droit et la pièce du support 5 qui lui est associée ne sont pas fixées rigidement au boîtier de la prothèse, comme dans la réalisation antérieure connue, mais sont solidarisées sur leur périphérie à un flasque d'extrémité d'un soufflet dont l'autre flasque d'extrémité est immobilisé par rapport au boîtier et/ou la cloison longitudinale 9 séparant, du point de vue de l'énergie pneumatique, le ventricule droit du centricule gauche.

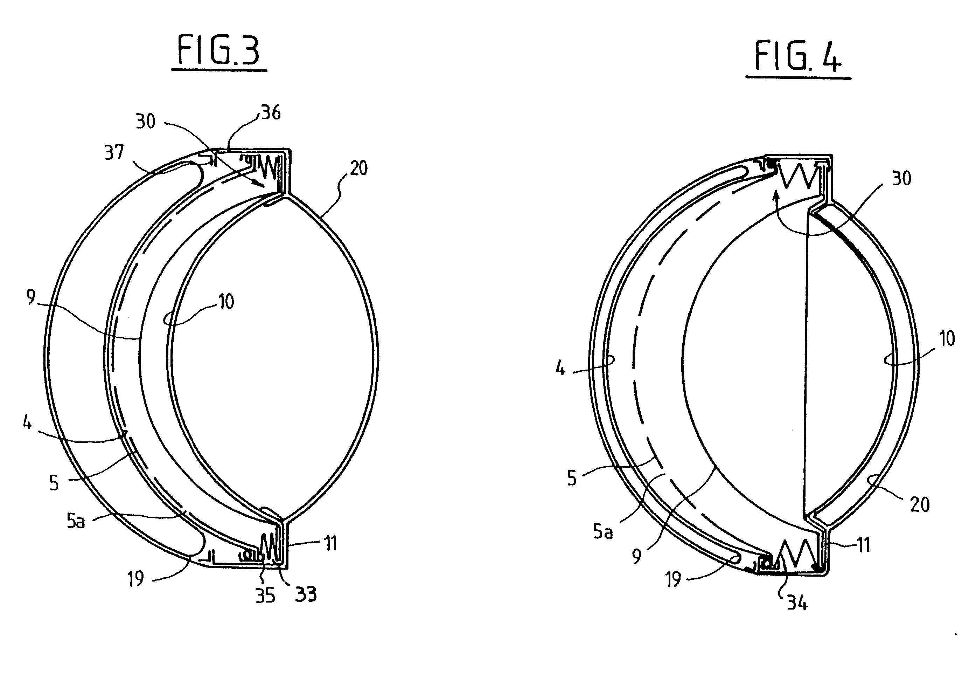

Dans une première réalisation, le soufflet 30 est selon l'invention, figures 1 à 4, en un matériau élastique, par exemple en élastomère (caoutchouc naturel ou néoprène) et du type à débattement parallèle, c'est-à-dire agencé de manière que ses spires 31, — comprimées en début de systole (figures 1 et 3) —, sont écartées les unes des autres sur la totalité de la périphérie de soufflet en fin de systole (figures 2 et 4). De façon plus précise, un des flasques d'extrémité 32 du soufflet 30 est serti par un anneau 33 sur la cloison 9, la membrane 10 et son support 11, tandis que son autre flasque d'extrémité 34 est serti à un anneau ou bague 35 sur la périphérie de la membrane 4 et de la pièce de support 5.

Des étriers de butée 36, solidaires de l'anneau 33 et avantageusement disposés dans le boîtier 1, 2 de la prothèse, de part et d'autre du plan de symétrie dudit boîtier, présentent à leurs extrémités distantes du flasque 32 des retours 37 avec lesquels est propre à coopérer l'autre flasque d'extrémité 34 du soufflet pour limiter l'amplitude de son débattement lors de la phase de systole. Au début de celle-ci, la condition de la prothèse est celle montrée sur les figures 1 et 3 ; l'introduction de fluide sous pression par la tubulure 16a provoque d'abord le déplacement de la membrane 4 et de la pièce de soutien 5 qui lui est associée, avec compression concomitante du sac à sang 19, jusqu'à butée du flasque 34 sur les arrêts 37 puis, à partir de cette position, une augmentation de la pression du fluide moteur entraîne l'élongation de la membrane 4 pour parachever la phase systolique du ventricule droit jusqu'à vidage total du sac à sang.

La décomposition de la systole en deux phases permet de limiter l'élongation de la membrane 4 à une valeur qui peut être de l'ordre de 7 à 10%, c'est-à-dire une valeur qui n'induit pas dans le matériau constitutif de la membrane des risques de micro-fissures ou analogues susceptibles d'en provoquer la destruction pour un nombre de cycles de fonctionnement inférieur à celui escompté. Nonobstant la valeur relativement faible de l'allongement de la membrane 4, le volume de sang expulsé à chaque pulsation est celui requis grâce à la phase initiale de compression du sac à sang lors du mouvement de débattement du soufflet 30.

Le dispositif moteur peut être alimenté, à partir des tubulures 16a et 16b, en phase ou en opposition de phase, ce dernier mode de fonctionnement autorisant un module énergétique de moindre puissance instantanée et permettant en outre de mieux régler, séparément, les pressions de commande du ventricule droit et du ventricule gauche.

Dans un second mode de réalisation, figures 5 à 7, le soufflet 40 associé à la membrane 4 et à sa pièce support 5 du ventricule droit est du type pendulaire et non plus à débattement parallèle comme dans la réalisation précédente. Il comprend alors des spires 41 susceptibles de s'écarter les unes des autres dans la zone de la pointe du ventricule droit de la prothèse et une charnière d'articulation 42 à l'opposé de la pointe de la prothèse, c'est-à-dire dans la zone de base de celle-ci. Comme dans la réalisation précédente, un flasque d'extrémité 43 du soufflet élastique est serti à la cloison 9, la membrane 10 et son support 11, à l'aide d'une bague ou anneau 44 maintenu dans les alvéoles du boîtier 1, 2 de la prothèse, non représentés, ou par des pattes 38, tandis que le flasque d'extrémité opposé 45 est serti, avec la membrane 4 et la pièce de support 5 dans une bague ou anneau 46. Des étriers de butée 47, munis de retour d'arrêts 48 avec lesquels est propre à coopérer le flasque 45, sont ici aussi prévus solidaires de l'anneau 44 dans l'espace ventriculaire droit pour limiter le mouvement pendulaire du soufflet 40. Dans une telle réalisation, dont le fonctionnement est pour le reste analogue à celui décrit ci-dessus, la présence d'une charnière d'articulation du soufflet à la base de la prothèse permet, lors de la première phase du mouvement systolique du ventricule droit, de provoquer d'abord le vidage de la pointe du sac à sang 19, qui est la plus distante de la charnière d'articulation, sans modifier sensiblement, simultanément, la forme générale de l'espace ventriculaire de la zone valvulaire au cours de cette première phase de la systole. Au cours de la seconde phase, c'est-à-dire celle qui comporte une élongation de la membrane 4, la densité plus importante des perforations 5a de la pièce de support 5 vers la base de la prothèse déforme d'abord ladite membrane dans sa partie de pointe, avec pour conséquence une mise en vitesse du sang contenu dans le sac 19 de la pointe vers la base, favorable à la bonne exécution du phénomène circulatoire.

Dans l'une et l'autre réalisations décrites ci-dessus le flasque d'extrémité fixe est avantageusement serti comme montré sur la figure 6a, c'est-à-dire dans une bague ou anneau 33 ou 44 qui assure, simultanément, la fixation de la cloison 9, de la membrane 10 travaillant à la déformation et du support 11 de ladite membrane dans la partie 1, 2 du boîtier de la prothèse, ledit boîtier comportant en outre les pattes 38 de maintien latéral dudit anneau 33, 44 et/ou des butées 36, 47 par leurs retours.

Alors que dans les deux réalisations décrites ci-dessus les tubulures 16a et 16b de liaison du dispositif moteur à la source du fluide sous pression sont disposées parallèlement l'une à l'autre, il peut être avantageux,dans certaines réalisations, de prévoir des raccords concentriques et non plus parallèles. Dans une telle réalisation, figure 8, c'est le forage central 50 d'une pièce 51, placée à la pointe de la prothèse, qui joue le rôle de tubulure 16a, le rôle de la tubulure 16b étant assuré par une chambre annulaire 52 entourant le forage 50 et qui communique par un ou des passage(s) 53 avec l'espace limité par la cloison 9 et la membrane 10 travaillant à la déformation. Dans cette réalisation, le soufflet à débattement parallèle 30 ou le soufflet pendulaire 40 est disposé entre la partie 2 du boîtier de la prothèse et le corps 54 externe limitant la chambre 52, avec un montage des flasques fixe et mobile dudit soufflet analogue à celui décrit ci-dessus.

## Revendications

1. Prothèse cardiaque totale comportant un boîtier (1, 2) implantable dans la cavité péricardique et dont la géométrie reproduit de très près celle du coeur naturel avec un dispositif moteur à l'intérieur dudit boîtier comportant essentiellement deux membranes (4, 10) dont l'une (4) travaille à l'élongation dans un espace définissant le ventricule droit et dont l'autre (10) travaille à la déformation dans un espace définissant le ventricule gauche, avec des sacs à sang (19, 20) enfermés dans lesdits espaces ventriculaires droit (17) et gauche (18), propres à être reliés aux vaisseaux du réseau circulatoire d'un patient par des valves montées dans des orifices valvulaires (12, 13, 14, 15) ménagés dans le boîtier (1, 2) de la prothèse laquelle est actionnée par des moyens d'activation du dispositif moteur comme une source d'énergie pneumatique comportant des moyens à boucles d'asservissement pour la régulation du débit sanguin, caractérisée en ce que la membrane élastique (4) du ventricule droit et une pièce de support (5) qui lui est associée sont montées dans le boîtier (1, 2) de la prothèse à l'aide de moyens agencés de manière telle que le mouvement de ladite membrane (4) à l'élongation (systole) est décomposé en deux phases dont l'une est un déplacement sans allongement et dont l'autre, seulement, est accompagnée d'un allongement, tandis que le mouvement inverse (diastole) comprend d'abord une phase de rétraction suivie d'un déplacement sans modification de forme de ladite membrane (4) et de la pièce support qui lui est associée (5).

2. Prothèse cardiaque totale selon la revendication 1, caractérisée en ce que les moyens procurant la décomposition en deux phases du mouvement de la membrane (4) du ventricule droit comprennent au moins un soufflet (30,40) propre à être relié à la source d'énergie pneumatique et sur lequel sont fixées la membrane (4) et la pièce de support (5) qui lui est associée.

3. Prothèse cardiaque totale selon la revendication 2, caractérisée en ce que le soufflet (30) est du type à déplacement parallèle.

4. Prothèse cardiaque totale selon la revendication 2, caractérisée en ce que le soufflet (40) est du type à déplacement pendulaire.

5. Prothèse cardiaque totale selon la revendication 2, caractérisée en ce que le soufflet (30, 40) est réalisé en un matériau élastique, en ce que son extrémité distante de celle de fixation de la membrane (4) et de la pièce support (5) qui lui est associée est fixée par sertissage à une cloison étanche (9) séparant les espaces ventriculaires droit (17) et gauche (18) de la prothèse, et en ce que sur ladite cloison (9) est également fixée la membrane (10) travaillant à la déformation du ventricule gauche.

6. Prothèse cardiaque totale selon la revendication 2, caractérisée en ce que la phase de déplacement sans allongement de la membrane (4) travaillant à l'élongation du ventricule droit est limitée, à l'élongation de ladite membrane (systole), par butée sur un organe d'arrêt (36, 47) de l'extrémité mobile du soufflet sur laquelle est attachée ladite membrane (4) et la pièce de support (5) qui lui est associée.

7. Prothèse cardiaque totale selon la revendication 2, caractérisée en ce qu'elle comprend un lubrifiant sec ou liquide entre les sacs à sang (19, 20), le boîtier (1, 2) de la prothèse, les membranes (4, 10) du dispositif moteur et les moyens (30, 40) associés à la membrane du ventricule droit pour la décomposition du mouvement de celle-ci en deux phases distinctes.

8. Prothèse cardiaque totale selon la revendication 4, caractérisée en ce que la charnière d'articulation (42) du soufflet (40) est prévue dans la zone de base de la prothèse, à l'opposé de la pointe de celle-ci où sont logées les spires (41) du soufflet (40) de sorte que le vidage de la poche à sang (19) du ventricule droit débute par la pointe dudit sac, favorisant ainsi le processus circulatoire.

9. Prothèse cardiaque totale selon l'une quelconque des revendications 1 à 8, caractérisée en ce que la pièce de support (5) de la membrane (4) travaillant à l'élongation du ventricule droit présente une plus grande densité de perforations (5a) dans la zone adjacente à la pointe de la prothèse que dans le reste de ladite pièce de support (5).

10. Prothèse cardiaque totale selon l'une quelconque des revendications précédentes, caractérisée en ce que le soufflet (30, 40) est immobilisé à l'aide de pattes (38) et par un de ses flasques d'extrémité (32, 43) par rapport au boîtier (1, 2) de la prothèse ledit flasque (32, 43) étant serti à l'aide d'une bague ou anneau (33, 44) coopérant avec lesdites pattes (38) et en ce que son autre flasque d'extrémité (34, 45) est serti par une bague ou anneau (35, 46) sur la membrane (4) travaillant à l'élongation et la pièce support (5) qui lui est associée.

11. Prothèse cardiaque totale selon la revendication 6, caractérisée en ce que le débattement du soufflet (30, 40) est limité par des étriers (36, 47) solidaires de l'anneau fixe (33, 44) et logés dans l'espace ventriculaire droit (17) lesdits étriers étant munis de retours d'arrêts (37, 48) du flasque d'extrémité mobile du soufflet.

12. Prothèse cardiaque totale selon l'une quelconque des revendications précédentes, caractérisée en ce que la source d'énergie pneumatique est reliée au dispositif moteur par une pièce (51) ménageant des alimentations coaxiales du ventricule droit et du ventricule gauche, et en ce que le soufflet (30, 40) est placé à la base de la prothèse, entre une partie (2) du boîtier de celle-ci et ladite pièce (51) laquelle est placée à la pointe de la prothèse entre les parties fixe et mobile du soufflet (30, 40).

**Patentansprüche**

1. Herzvollprothese mit einem Gehäuse (1, 2), das in die Herzbeutelhöhle implantiert werden kann und dessen Geometrie jene des natürlichen Herzens sehr nahe nachbildet, mit einer Bewegungseinrichtung im Inneren des Gehäuses, das im wesentlichen zwei Membrane (4, 10) enthält, bei welchen eine (4) mittels Elongation in einem Raum arbeitet, welcher die rechte Kammer definiert und bei welchen die andere (10) mittels Deformierung in einem Raum arbeitet, welcher die linke Kammer definiert, wobei Blutsäcke (19, 20) in den rechten (17) und linken (18) Ventrikelräumen eingeschlossen sind, die mit den Gefäßen des Kreislaufes eines Patienten über Klappen verbunden werden können, die in den Klappenöffnungen (12, 13, 14, 15) angebracht sind, die in dem Gehäuse (1, 2) der Prothese angeordnet sind, die durch Aktivierungsmittel der Bewegungseinrichtung, wie eine pneumatische Energiequelle mit Regelkreismitteln für die Regelung des Blutdurchflusses betätigt wird, dadurch gekennzeichnet, daß die elastische Membran (4) der rechten Kammer und ein Trägerteil (5), der ihr zugeordnet ist, in dem Gehäuse (1, 2) der Prothese mit Hilfe von Mitteln angebracht sind, die so angeordnet sind, daß die Bewegung der Membran (4) mittels Elongation (Systole) in zwei Phasen zerlegt ist, bei welchen die eine eine Verschiebung ohne Dehnung ist und bei welchen nur die andere von einer Dehnung begleitet ist, wogegen die umgekehrte Bewegung (Diastole) zuerst eine Zusammenziehphase gefolgt von einer Verschiebung ohne eine Formveränderung der Membran (4) und des ihr zugeordneten (5) Trägerteiles umfaßt.

2. Herzvollprothese nach Anspruch 1, dadurch gekennzeichnet, daß die Mittel, welche für die Zerlegung der Bewegung der Membran (4) der rechten Kammer in zwei Phasen sorgen, zumindest einen Balg (30, 40) aufweisen, der mit der pneumatischen Energiequelle verbunden werden kann und auf welchem die Membran (4) und der ihr zugeordnete Trägerteil (5) befestigt sind.

3. Herzvollprothese nach Anspruch 2, dadurch gekennzeichnet, daß der Balg (30) der Parallelverschiebung-Art angehört.

4. Herzvollprothese nach Anspruch 2, dadurch gekennzeichnet, daß der Balg (40) der Pendelverschiebung-Art angehört.

5. Herzvollprothese nach Anspruch 2, dadurch gekennzeichnet, daß der Balg (30, 40) aus einem elastischen Material ausgeführt ist, daß sein entferntes Ende von demjenigen einer Befestigung der Membran (4) und des ihr zugeordneten Trägerteiles (5) durch Pressen zu einer dichten Trennwand (9) befestigt ist, welche die rechten (17) und linken (18) Ventrikelräume der Prohtese trennt und daß auf der Trennwand (9) auch die Membran (10) der linken Kammer befestigt ist, die mittels Deformation arbeitet.

6. Herzvollprothese nach Anspruch 2, dadurch gekennzeichnet, daß die Verschiebungsphase ohne Dehnung der Membran (4) der rechten Kammer die mittels Elongation arbeitet durch einen Anschlag auf einem Halteteil (36, 47) des beweglichen Endes des Balges auf die Elongation der Membran (Systole) begrenzt ist, auf welchem die Membran (4) und der ihr zugeordnete Trägerteil (5) befestigt ist.

7. Herzvollprothese nach Anspruch 2, dadurch gekennzeichnet, daß die zwischen den Blutsäcken (19, 20), dem Gehäuse (1, 2) der Prothese, den Membranen (4, 10) der Bewegungseinrichtung und den der Membran der rechten Kammer zugeordneten Mitteln (30, 40) für die Zerlegung der Bewegung dieser in zwei unterschiedliche Phasen ein trockenes oder ein flüssiges Schmiermittel aufweist.

8. Herzvollprothese nach Anspruch 4, dadurch gekennzeichnet, daß die Gelenkverbindung (42) des Balges (40) in dem Basisbereich der Prothese, auf der gegenüberliegenden Seite der Spitze vorgesehen ist, wo die Windungen (41) des Balges (40) so aufgenommen sind, daß das Entleeren des Blutsackes (19) der rechten Kammer durch die Spitze des Sackes beginnt, wodurch der Kreislaufprozeß gefördert wird.

9. Herzvollprothese nach irgendeinem der Ansprüche 1 bis 8, dadurch gekennzeichnet, daß der Trägerteil (5) der Membran (4) der rechten Kammer, die mittels Elongation arbeitet, eine viel größere Dichte von Perforationen (5a) in dem zu der Spitze der Prothese benachbarten Bereich aufweist, als in dem Rest des Trägerteiles (5).

10. Herzvollprothese nach irgendeinem der vorgehenden Ansprüche, dadurch gekennzeichnet, daß der Balg (30, 40) mit Hilfe von Laschen (38) und durch einen seiner Endflansche (32, 43) bezüglich des Gehäuses (1, 2) der Prothese angehalten ist, wobei der Flansch (32, 43) mit Hilfe einer Büchse oder eines Ringes (33, 44) gefaßt ist, der mit den Laschen (38) zusammenwirkt und daß sein anderer Endflansch (34, 45) durch eine Hülse oder einen Ring (35, 46) auf der Membran (4), die mittels Elongation arbeitet und dem Trägerteil (5), der ihr zugeordnet ist, gefaßt ist.

11. Herzvollprothese nach Anspruch 6, dadurch gekennzeichnet, daß der Anschlag des Balges (30, 40) durch Bügel (36, 47) begrenzt ist, die mit dem festen Ring (33, 44) verbunden sind und in dem rechten (17) Ventrikelraum aufgenommen sind, wobei die Bügel mit Halterückläufen (37, 48) des beweglichen Endflansches des Balges versehen sind.

12. Herzvollprothese nach irgendeinem der vorgehenden Ansprüche, dadurch gekennzeichnet, daß die pneumatische Energiequelle mit der Bewegungseinrichtung durch einen Teil (51) verbunden ist, der eine koaxiale Versorgung der rechten Kammer und der linken Kammer durchführt und daß der Balg (30, 40) an der Basis der Prothese zwischen einem Teil (2) ihres Gehäuses und dem Teil (51) angebracht ist, der an der Spitze der Prothese zwischen den festen und beweglichen Teilen des Balges (30, 40) angebracht ist.

## Claims

1. A total heart prosthesis comprising a case (1, 2) implantable in the pericardial cavity and the geometry of which is very similar to that of the natural heart with, a motor device, inside this case, comprising essentially two membranes (4, 10) one of which (4) works by elongation within a space defining the right ventricle and the other one (10) of which works by deformation within a space defining the left ventricle, with blood bags (19, 20) enclosed in said right (17) and left (18) ventricular spaces, adapted to be connected to the vessels of the circulatory system of a patient by means of valves mounted in valvular orifices (12, 13, 14, 15) formed in the case (1, 2) of the prosthesis which is actuated by means activating the motor device such as a pneumatic energy source comprising servo loop means for regulating the blood flow, characterized in that the elastic membrane (4) of the right ventricle and a support part (5) which is associated therewith are mounted in the case (1, 2) of the prosthesis by means so adapted that the movement of said membrane (4) during elongation (systole) is broken down into two phases, one of which is a displacement without elongation and the other one, only, is accompanied by an elongation whereas the reverse movement (diastole) comprises first a retraction phase followed by a displacement without modification of the shape of said membrane (4) and of the support part associated therewith (5).

2. The total heart prosthesis as claimed in Claim 1, wherein the means for breaking the movement of the membrane (4) of the right ventricle down into two phases comprise at least one bellows (30, 40) adapted to be connected (4) to the pneumatic energy source and on which membrane (4) and the support part (5) associated therewith are fixed.

3. The total heart prosthesis as claimed in Claim 2, wherein the bellows (30) is of the parallel displacement type.

4. The total heart prosthesis as claimed in Claim 2, wherein the bellows (40) is of the pendular displacement type.

5. The total heart prosthesis as claimed in Claim 2, wherein the bellows (30, 40) is made from an elastic material, wherein its end distant from that by which the membrane (4) and the support part (5) associated therewith are fixed is fixed by crimping to a sealed dividing wall (9) separating the right (17) and left (18) ventricular spaces of the prosthesis, and wherein on said dividing wall (9) is also fixed the membrane (10) working under deformation of the left ventricle.

6. The total heart prosthesis as claimed in Claim 2, wherein the displacement phase without elongation of the membrane (4) working under elongation of the right ventricle is limited, during elongation of said membrane (systole) by abutting against a stop member (36, 47) on the mobile end of the bellows to which said membrane (4) and the support part (5) associated therewith are fixed.

7. The total heart prosthesis as claimed in Claim 2, characterized in that it comprises a dry or liquid lubricant between the blood bags (19, 20), the case (1, 2) of the prosthesis, the membranes (4, 10) of the motor device and the means (30, 40) associated with the membrane of the right ventricle for breaking down the movement thereof into two distinct phases.

8. The total heart prosthesis is as claimed in Claim 4, wherein the hinge (42) of the bellows (40) is provided in the base zone of the prosthesis, opposite the tip thereof where the turns (41) of the bellows (40) are housed so that emptying of the blood (19) pocket of the right ventricle begins by the tips of said bag, thus promoting the circulatory process.

9. The total heart prosthesis as claimed in any one of Claims 1 to 8, wherein the support part (5) of the membrane (4) working under elongation of the right ventricle has a greater density of perforations (5a) in the zone adjacent the tip of the prosthesis than in the rest of said support part (5).

10. The total heart prosthesis as claimed in any one of the preceding claims, wherein the bellows (30, 40) is immobilized by means of lugs (38) and by one of its end flanges (32, 43) with respect to the case (1, 2) of the prosthesis, said flange (32, 43) being crimped by means of a band or ring (33, 44) cooperating with said lugs (38) and its other end flange (34, 45) is crimped by a band or ring (35, 46) on the membrane (4) working under elongation and the support part (5) which is associated therewith.

11. The total heart prosthesis as claimed in Claim 6, wherein the displacement of the bellows (30, 40) is limited by stirrups (36, 47) fixed to the fixed ring (33, 44) and housed in the right ventricular space (17), said stirrups being provided with bends (37, 48) for arresting the mobile end flange of the bellows.

12. The total heart prosthesis as claimed in any one of the preceding claims, wherein the pneumatic energy source is connected to the motor device by a piece (51) providing coaxial feeding of the right ventricle and the left ventricle, and wherein the bellows (30, 40) are placed at the base of the prosthesis, between a portion (2) of the case thereof and said piece (51) which is placed at the tip of the prosthesis between the fixed and mobile portions of the bellows (30, 40).

FIG.1

FIG.2

EP 0 300 012 B1

## FIG.3

## FIG. 4

EP 0 300 012 B1

FIG.5

FIG.6

FIG.6a

FIG.7

FIG.8

FIG.9

EP 0 300 012 B1